# EUROPEAN PATENT APPLICATION

(11) **EP 4 755 317 A1**
(43) Date of publication of application: **10.06.2026**
(21) Application number: 25218197.9
(22) Date of filing: 25.11.2025
(51) Int. Cl.: A61B 10/00

(54) **A DEVICE FOR COLLECTING BIOLOGICAL MATERIAL SAMPLES FROM PATIENT S NASAL CAVITY**

(30) Priority: 03.12.2024 PL 45044024
(71) Applicant: Warszawski Uniwersytet Medyczny, 02-091 Warszawa (PL)
(72) Inventor: Krzych-Falta, Edyta, 03-358 Warszawa (PL)
(74) Representative: Patpol Kancelaria Patentowa Sp. z o.o.

(57) **Abstract**

The invention provides a device for collecting biological material samples from the nasal cavity (1) of a patient, to be coupled with a syringe (2) and a test tube (3). The device is characterized in that it comprises: a body (4) with an opening (5) for the test tube (3) to be coupled from below, and a nasal rinser (6) connected to the body (4) in the vicinity of the opening (5) and protruding upwardly, the nasal rinser (6) being open at the bottom for the syringe (2) to be inserted from below, and having spraying openings (6a) at the top, for spraying fluid into the nasal cavity (1) of the patient, and a sealing cap (7) covering the body (4) from above and overlapping the nasal rinser (6), with respective through holes (8, 9) corresponding to the opening (5) and the nasal rinser (6).

## Description

The invention provides a device for collecting biological material samples from the patent's nasal cavity, to be coupled with a syringe and a test tube. The device is useful for collecting material in the form of nasal washings, in order to perform immunoenzymatic tests under the conditions of intranasal provocation assay with an allergen or during the pollen season or exposure to year-round allergens.

A vast portion of the literature in the art is dedicated to the opportunities provided by detection of inflammatory response biomarkers using immunoenzymatic methods in biological material, including nasal washings. Mainly, this applies to differential diagnostics of rhinitis. A particular variety of the latter is a local allergy-based rhinitis the diagnosis of which is mainly based on clinical history and allergen intranasal provocation test performed under controlled conditions. During the test, the allergen is deposited in the area of the lower nasal concha head (using a standardized atomiser) to trigger a cascade of events known as a "local anaphylactic reaction within the mucosa of the nasal cavity." This induces an IgE-dependent or eosinophil response which causes release of inflammatory cells (histamine, tryptase: α-tryptase and β-tryptase, responsible for the activity of mast cells, eosinophil cationic protein (ECP), as a standardized marker protein for the eosinophilic activity of the mucosa of the nasal cavity, myeloperoidase (MPO) or immunoglobulins: sIgE, IgA, IgG, IgG4, as an indicator for monitoring effectiveness of the therapy used).

The main problem in standardization of the techniques/equipment used for collecting biological material from the nasal cavity is the great number of technical solutions and quite large discrepancy in the data/results obtained (as shows the literature review). This causes a necessity of lowering of the detection rate of sIgE in immunoenzymatic tests and this directly induces difficulties in evaluation of the method specificity and sensitivity. Biological material is collected from the nasal cavity by:
- mechanical evacuating (blowing out) or spontaneous downflow of secretion material which is then transported, via a Pasteur pipette, to an Eppendorf test tube comprising a solid substance and dilution buffer. A disadvantage of this technique is that too little secretion is obtained for further testing;
- aspiration of the secretion material by means of a silicone suction tube, connected to a vacuum pump and a test tube for nasal washings with phosphate-buffered saline. A technical problem in this technique is a considerably high dilution factor and too little amount of secretion material from the nasal cavity. Moreover, mechanical removal of the secretion material increases the risk of non-specific response of the nasal cavity mucosa;
- irrigation of the nasal cavity with 10 ml of saline buffer during which the secretion material flows down physiologically onto a Petri dish or with the use of a catheter which is inserted into the nasal cavity. Similarly as above, collection of biological material poses a risk of nasal washings high dissolution factor, and this significantly affects the test result;
- rinsing of the nasal cavity; for this purpose a special device is used - a plastic container with a nasal adapter through which 10 ml of saline is applied. A disadvantage of the test is a high dissolution factor of the collected material and a considerable loss of the latter;
- a spraying pump by means of which an amount of isotonic or hypertonic solution of sodium chloride (1 to 5 ml of 0,9-1,8% NaCl) is fed. Diluted material is stored for incubation and inflammatory response biomarkers are detected the in immunoenzymatic tests. In the group of the above mentioned techniques, this method enables obtaining of the sufficient amount of biological material to enable detection of sIgE in immunoenzymatic tests. Literature containing analysis of specificity and sensitivity of the tool dates back to the 1990s;
- layers to absorb the secretion material, e.g., cotton, filter, rubber foam, placed between the septum and the inferior nasal concha and left for about ten minutes and then centrifuged to extract the nasal washings. A disadvantage of this test is a risk of non-specific response to the absorbent layer positioned in the area of the inferior nasal concha head.

A specific alternative for the above mentioned techniques involves direct positioning of sIgE (ImmunoCAP^{®}) paste comprising allergens under in situ conditions and for the time of about five minutes, between the nasal septum and the head of the inferior nasal concha. An important disadvantage of the method is a risk of inducing a positive nasal cavity mucosa response to the applied sIgE paste. Another method is an intranasal Schirmer test which is used to evaluate the amount and humidity of the nasal cavity secretion, as commonly used in rhinology.

The subject invention provides a device for collecting biological material samples from the patient's nasal cavity, to be coupled with a syringe and a test tube. The invention is characterized in that it comprises
- a body with an opening for a test tube to be coupled from below, and
- a nasal rinser, connected to the body in the vicinity of the opening and protruding upwardly, the nasal rinser being open at the bottom for a syringe to be inserted from below, and having spraying openings at the top to spray fluid into the nasal cavity of the patient, and
- a sealing cap covering the body from above and overlapping the nasal rinser, with respective through holes corresponding to the opening and the nasal rinser.

Preferably, the device is made integral.

Preferably, the device is made by 3D printing.

Preferably, the opening is threaded.

Preferably, the sealing cap has an external edge protruding outwardly.

Preferably, the body is made of polyetheretherketone.

Preferably, the sealing cap is made of polypropylene filaments.

The subject-matter of the invention is shown in its embodiments in the drawings, and fig. 1 shows a general view of a device, with a syringe and a test tube coupled therewith, when used in a patient for collecting a sample, i.e., showing the operation principle of the device, fig. 2 shows a view of a device with a test tube coupled therewith, with constructive details of the device, and fig. 3 shows an enlarged device alone to illustrate details in an enlarged view, along with a cross-section.

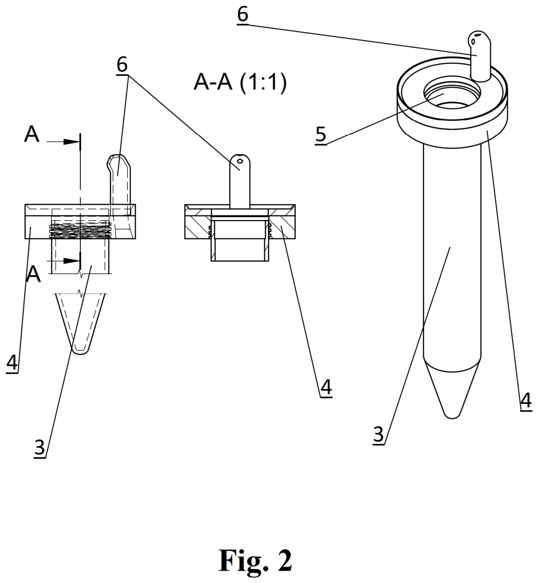

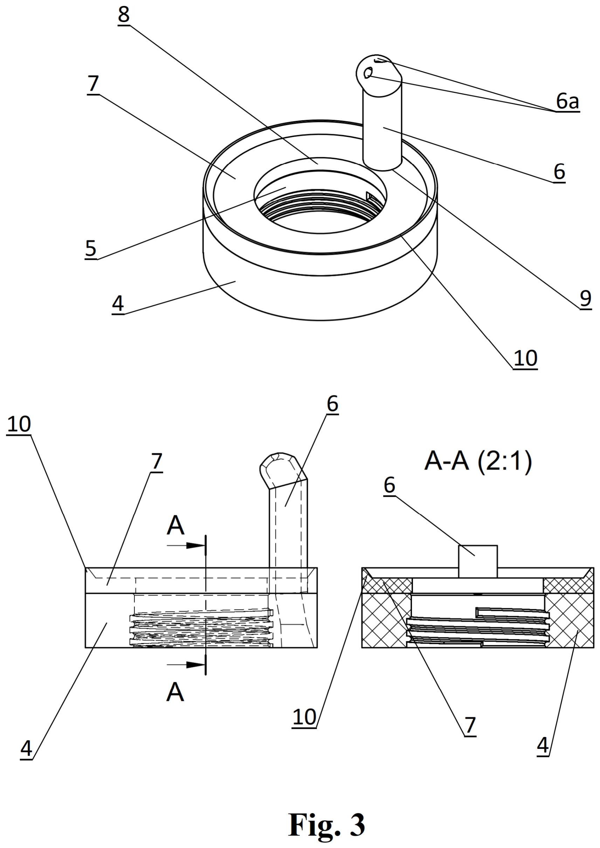

As shown in the drawings, the device according to the invention comprises a body 4 with an opening 5 for a test tube 3 to be coupled from below, and a nasal rinser 6 connected to the body 4 in the vicinity of the opening 5 and protruding upwardly. The nasal rinser 6 is open at the bottom for a syringe 2 to be inserted from below, and has spraying openings 6a at the top for spraying fluid into the nasal cavity 1 of the patient. From above, the body 4 is covered with a sealing cap 7, the cap 7 overlapping the nasal rinser 6. For this purpose, the cap 7 has respective through holes 8, 9 corresponding to the opening 5 and the nasal rinser 6. The device may be easily coupled with a syringe 3 (syringe end is inserted into the nasal rinser 6 which is open at the bottom), in order to spray a suitable solution into the nasal cavity 1 of the patient to cause secretion. The solution is then sprayed via the spraying openings 6a. The device may be also easily coupled with a test tube 3 which may be positioned within the opening 5. Diverse solutions may be used to prevent the test tube 3 from falling off from the opening 5. The best way involves a thread in the opening 5 (such embodiment is shown in figs. 2-3) and the opening 5 may be engaged with the corresponding thread on the test tube 3. Nevertheless, a person skilled in the art will note that this may be achieved in other ways, e.g., by inserting the test tube 3 with interference fit - for this purpose, e.g., an elastic insert/gasket may be used in the opening 5 or at the edge of the test tube 3, or the body may be made of some elastic material.

When a sample of material is collected, the patient has to maintain his/her head in a suitable position (to keep the device and the test tube vertically). Upon spraying of the solution by means of a syringe 2 and a nasal rinser 6, the secretion material is expected to flow down directly into the test tube 3 via the opening 5. The purpose of the sealing cap 7, in this situation, is solely to seal the connections between the individual components of the device. On the other hand, it is known that maintaining of such proper position of the patient's head is often impossible (in particular for children or persons with impaired mobility, e.g., elderly persons), and therefore, most preferably, the sealing cap 7 has an outer edge 10 protruding upwardly, as shown in the embodiments in the drawings, but this feature is not indispensable for the device to operate properly when the patient is an adult with average mobility.

In a preferable embodiment, the device is made integral (i.e., all its components constitute one element), nevertheless a person skilled in the art will note that it may be also made as separate components to be combined together at a further stage.

In a preferable embodiment, the device is made by 3D printing, most preferably, as shown, as an integral component, but with this technique the device also may be made as individual components to be combined together at a further production stage.

In a preferable embodiment, in particular capable to be made by 3D printing, the body 4 is made of polyetheretherketone (PEEK), and the sealing cap 7 is made of polypropylene (PP) filaments. In this arrangement, the cap 7 is made as a separate element and is applied onto the body from above.

The plastic body 4 has an advantage that it may be repeatedly used and sterilized (gas sterilization). It forms a kind of a framework for the device and stabilizes, in a way, the standard test tube 3 positioned in the lower portion and used for centrifugation of the secretion material from the supernatant under laboratory conditions, as well as the nasal rinser 6. In turn, the cap 7 made of an elastic material prevents loss of the obtained nasal washings.

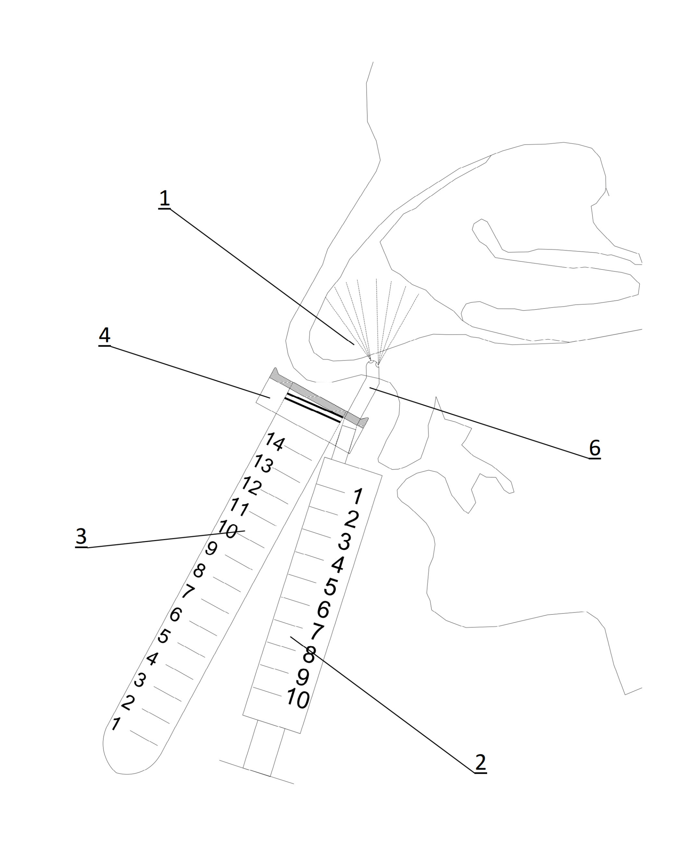

Through the nasal rinser 6, an isotonic saline solution is fed, e.g., in the amount of 5,2 ml. The device is laterally coupled with a standard syringe 2, most preferably having a volume of 10 ml (5 ml volume is too small, while 20 ml is cumbersome and the application is difficult due to the high piston resistance), via which, thanks to the stabilizing body 4, application of the rinsing substance for the nasal cavity 1 is possible. Rinsing of the nasal cavity 1 lasts in average 1,5 minutes with the patient's head inclined forwardly (Fig. 1). The secretion material gravitationally flows down directly into a connected test tube 3 (this may be a standard test tube used in laboratories and used further on for centrifugation and for immunoenzymatic assays, and thus there is no need to collect the material or transfer it separately to a test tube). The tested subject is recommended to breathe through his/her mouth at that time. The risk of aspiration of the secretion material into the nasal cavity 1 is minimal due to the appropriate amount of fluid for rinsing the nasal cavity 1.

The advantages of the device, over the known prior art, are as follows:
- minimized risk of loss of the collected material in the form of nasal washings,
- collecting of biological material of good quality (absence of contamination, low dilution level),
- the device is easy to be manufactured,
- low manufacturing costs of the device,
- possibility of repeated use and sterilization,
- possibility of manufacturing of device in variants with different sizes (suitable tor patients of different age).

It will be clear that the invention is not limited to the above described embodiments and the features indicated in the claims may be used in in any combination proper for the specific application of the solution.

## Claims

1. A device for collecting biological material samples from the nasal cavity (1) of a patient, to be coupled with a syringe (2) and a test tube (3), **characterized in that** it comprises
- a body (4) with an opening (5) for the test tube (3) to be coupled from below, and
- a nasal rinser (6) connected to the body (4) in the vicinity of the opening (5) and protruding upwardly, the nasal rinser (6) being open at the bottom for the syringe (2) to be inserted from below, and having spraying openings (6a) at the top to spray fluid into the nasal cavity (1) of the patient, and
- a sealing cap (7) covering the body (4) from above and overlapping the nasal rinser (6), with respective through holes (8, 9) corresponding to the opening (5) and the nasal rinser (6).

2. The device according to claim 1, **characterized in that** it is made integral.

3. The device according to claim 1 **characterized in that** it is made via 3D printing.

4. The device according to claim 1, **characterized in that** the opening (5) is threaded.

5. The device according to claim 1, **characterized in that** the sealing cap (7) has an outer edge (10) protruding upwardly.

6. The device according to claim 1, **characterized in that** the body (4) is made of polyetheretherketone.

7. The device according to claim 1, **characterized in that** the sealing cap (7) is made of polypropylene filaments.
